(19) Europäisches Patentamt / European Patent Office / Office européen des brevets

(11) **EP 4 469 201 B1**

(12) **EUROPEAN PATENT SPECIFICATION**

(45) Date of publication and mention
of the grant of the patent:
**29.10.2025 Bulletin 2025/44**

(21) Application number: **23701780.1**

(22) Date of filing: **26.01.2023**

(51) International Patent Classification (IPC):
**B01J 23/10** (2006.01)    **B01J 37/00** (2006.01)
**C07C 319/00** (2006.01)    **C07C 323/00** (2006.01)

(52) Cooperative Patent Classification (CPC):
(C-Sets available)
**B01J 23/10; B01J 37/0063; C07C 319/20**    (Cont.)

(86) International application number:
**PCT/EP2023/051918**

(87) International publication number:
**WO 2023/144265 (03.08.2023 Gazette 2023/31)**

(54) **GRANULAR CATALYST FOR THE HYDROLYSIS OF AMINO NITRILES AND AMINO AMIDES TO AMINO ACIDS OR DERIVATIVES THEREOF**

KÖRNIGER KATALYSATOR FÜR DIE HYDROLYSE VON AMINONITRILEN UND AMINOAMIDEN ZU AMINOSÄUREN ODER DERIVATEN DAVON

CATALYSEUR GRANULAIRE POUR L'HYDROLYSE DES AMINO NITRILES ET DES AMINO AMIDES EN ACIDES AMINÉS OU LEURS DÉRIVÉS

(84) Designated Contracting States:
**AL AT BE BG CH CY CZ DE DK EE ES FI FR GB
GR HR HU IE IS IT LI LT LU LV MC ME MK MT NL
NO PL PT RO RS SE SI SK SM TR**

(30) Priority: **28.01.2022 EP 22153798**

(43) Date of publication of application:
**04.12.2024 Bulletin 2024/49**

(73) Proprietor: Evonik Operations GmbH
**45128 Essen (DE)**

(72) Inventors:
• **BILZ, Jürgen**
**63579 Freigericht (DE)**
• **FISCHER, Achim**
**63773 Goldbach (DE)**
• **GEIST, Lucas**
**63579 Freigericht (DE)**
• **HARTMANN, Michael**
**97773 Aura (DE)**
• **REUS, Christian**
**63579 Freigericht (DE)**
• **WILZ, Frank**
**63755 Alzenau (DE)**

(74) Representative: **Evonik Patent Association
c/o Evonik Industries AG
IP Management
Postcode 84/339
Rodenbacher Chaussee 4
63457 Hanau (DE)**

(56) References cited:
**WO-A1-2020/161067    WO-A1-2020/249495**

(52) Cooperative Patent Classification (CPC): (Cont.)

C-Sets
**C07C 319/20, C07C 323/58;**
**C07C 319/20, C07C 323/60**

**Description**

The present invention relates to a granular catalyst for the hydrolysis of amino nitriles and/or amino amides to amino acids or derivatives thereof, a process for the preparation of this catalyst and a method for the preparation of an amino acid or derivative thereof comprising the step of contacting a solution or suspension comprising an amino nitrile and/or an amino amide with water in the presence of a catalyst according to the present invention or in the presence of a catalyst obtained by a process according to the present invention.

Amino acids have a fundamental importance in animal nutrition due to their function as protein building blocks for the nutrition of animals and humans. Feed stuffs are therefore additionally enriched with amino acids such as D,L-methionine, which increases their nutritional value. D,L-methionine is an essential amino acid that must be ingested with food. As a feed additive, it contributes to an efficient, healthy and environmentally friendly diet of farm animals, especially poultry and pigs. It is therefore also an important building block when it comes to the sustainable supply of a growing world population with animal protein. Therefore, a cost-effective synthesis process for D,L-methionine, which is also well suited for large-scale industry, is of great importance.

On an industrial scale, methionine is chemically produced via the Bucherer-Bergs reaction, which is a variant of the Strecker synthesis. The starting materials for this reaction are 3-methylmercaptopropionaldehyde (MMP), which is produced from the reaction of 2-propenal with methyl-mercaptan, hydrocyanic acid (hydrogen cyanide), ammonia and carbon dioxide. Said starting materials are reacted to give 5-(2-methylmercaptoethyl)-hydantoin (methionine hydantoin), which is hydrolyzed to give methionate. Hydrolysis of the methionine hydantoin requires harsh conditions and stoichiometric amounts of a base, usually sodium hydroxide, potassium hydroxide or potassium carbonate. Hence, the hydrolysis of the methionine hydantoin typically gives the sodium or potassium salt of methionine (sodium or potassium methionate). According to a known method, the methionine is released from the sodium or potassium methionate by neutralizing this salt with sulfuric acid. The thus formed neutral methionine precipitates from the solution and can be easily filtered off from the solution. The sodium or potassium sulphate produced as a by-product must be recycled or otherwise disposed of.

The so-called Degussa potassium carbonate circulation process is disclosed in US 5,770,769. Here, methionine is finally released from its potassium salt by treating the hydrolysate with carbon dioxide, wherein the methionine precipitate can be filtered off from the mother liquor containing potassium carbonate and potassium bicarbonate. The latter can be recovered, but this requires a circuit with a large amount of saline. In addition, the methionine hydantoin formation and hydrolysis require harsh and energy-intensive conditions, for example temperatures of up to 200 °C in the hydrolysis of the hydantoin. Hence, there was still a need for an industrially feasible process that either does not have these disadvantages at all or only to a small extent.

One approach to avoid the said disadvantage is the preparation of methionine from 2-amino-4-(methylthio) butane nitrile (methionine nitrile; MMP aminonitrile; MMP-AN) via 2-amino-4-(methylthio)butane amide (methionine amide) in the presence of various metal oxide catalysts.

WO 2001/060788 A1 discloses an overall process for the preparation of methionine in which methionine nitrile, is prepared from 2-hydroxy-4-(methylthio)butane nitrile (MMP cyanohydrin, MMP-CN) and ammonia, is hydrolyzed by alkali hydroxide in the presence of acetone to methionine amide, which then is further hydrolyzed to ammonium methionate with the help of a titanium-containing catalyst, in particular titanium dioxide. Ammonia is released and expelled so that solid methionine is obtained as the end-product. Despite a reaction temperature of 100 °C in the last step, the conversion is not complete, and the description does not allow conclusions to be drawn about the actual selectivity and yield of methionine in the reaction, so that undesirable residues of methionine amide remain in the final product (Table 1, WO 2001/060788 A1).

JP03-093753 A discloses a method for hydrolysis of amino acid nitriles to amino acid amides or amino acids. A corresponding amino acid nitrile is hydrolyzed to methionine amide with at least one equivalent, preferably ten equivalents of water in the presence of 0.01 - 0.5 mole equivalents (mol eq.) of a metal oxide from the group of zirconium oxide, titanium oxide, niobium oxide, titanium oxide tungsten oxide, titanium oxide tin oxide and titanium oxide zinc oxide at temperatures of 100 - 200 °C. The subsequent hydrolysis step to methionine is carried out in the presence of a metal oxide from the group of zirconium oxide, titanium oxide, niobium oxide, titanium oxide tungsten oxide, titanium oxide tin oxide and titanium oxide zinc oxide. The yield for methionine with a zirconium oxide-titanium oxide-niobium oxide catalyst is at best 94% (Example 1, JP03-093753 A). However, the disadvantage here is the relatively high demand for catalyst, namely 100 g catalyst powder per 10 g amino acid amide as starting material (Example 1, JP03-093753 A).

JP03-093754 A also discloses a method for hydrolysis of amino acid nitriles to amino acid amides or amino acids. A corresponding amino acid nitrile is hydrolyzed with at least one equivalent, preferably ten equivalents of water in the presence of 0.01 - 0.5 mol eq. of a metal oxide from the group of titanium oxide zirconium oxide, titanium oxide aluminum oxide, titanium oxide niobium oxide, titanium oxide tungsten oxide, titanium oxide tin oxide, titanium oxide zinc oxide at temperatures of 50 - 220 °C. Hydrolysis of amino acid nitrile is carried out in the presence of 0.01 - 0.5 mol eq. of a metal oxide from the group of titanium oxide-zirconium oxide, titanium oxide-aluminum oxide, titanium oxide-zinc oxide. However, in the hydrolysis of methionine amide to methionine, the methionine yield was only 93% (with titanium oxide zirconium oxide) and 94% (with titanium oxide niobium oxide) according to Examples 5 and 1 (JP03-093754 A), and the catalyst consumption was quite high (0.34 to 0.68 g catalyst per g of the amide).

According to the above-mentioned publications, so far mainly catalysts based on titanium dioxide have been used. The $ZrO_2$ catalysts used in the papers discussed showed significantly worse performance and were not studied for their crystal modification or structure-property relationships. It is also known from titanium dioxide catalysts that only those with an anatase crystal modification catalyze the reaction of methionine amide to methionine. $TiO_2$ in rutile modification, on the other hand, shows no catalytic effect.

EP3199519 A1 discloses a process for preparing methionine in which 2-amino-4-(methylthio)butane nitrile and water are brought into contact with each other in the presence of cerium-containing oxide catalysts. The cerium-containing catalyst is either a $CeO_2$ catalyst or a $CeO_2$-$ZrO_2$ mixed oxide catalyst, each in the form of a fine powder. The yields of methionine range from 60.9% to 97.0%. The reaction is carried out according to the examples at temperatures of 60 to 100 °C and residence times of about 1 to 2 hours.

In addition, WO 2018/021338 discloses a process for preparing an alpha-amino acid by reacting the corresponding alpha-amino acid amide with water in the presence of a zirconium compound and at least one other metal such as hafnium or cerium. Herein, in particular, the hydrolysis of methionine amide to methionine is disclosed using a zirconium-containing complex metal oxide catalyst containing Hf, Ce, Y or Ti (Examples 4, 5 and comparative examples 2, 3). However, this document does neither disclose the $ZrO_2$ content of the complex metal oxide catalyst nor the particle size or any other feature regarding the morphology of the catalyst. Regarding the continuous process, the document merely discloses that a continuous tank reactor and a tubular reactor can be used, also known as a continuous stirred tank reactor (CSTR) and plug flow reactor (PFR). However, both types are slurry reactors which, due to their complicated operation, are not suitable for a large-scale process such as the production of methionine. The examples also do not offer a process that can be used in continuous production, since they are all carried out in batches, e.g., in a pressure vessel made of steel in which a stirrer is inserted (Example 1).

The two more recent applications WO 2020/161067 A1 and WO 2020/161074 A1 disclose further developments in the field of $CeO_2$ and $ZrO_2$ comprising catalysts for the hydrolysis of methionine nitrile to methionine.

WO 2020/161074 A1 discloses a particulate catalyst with a content of 60.0 to 99.5 wt.% $ZrO_2$, which is stabilized with an oxide of hafnium and at least one oxide of element M with M being Ce, Si, Ti or Y, with a median x50 of 0.8 to 9.0 mm. However, the catalyst can only be used for hydrolysis of already formed methionine amide to methionine and not for the complete hydrolysis to methionine starting from methionine nitrile. This makes the said catalyst disadvantageous for the methionine production.

WO2020/161067 A1 discloses a $CeO_2$-based catalyst, with a $CeO_2$ content of from 50 to 100 wt.-%, for the direct production of methionine by hydrolysis of methionine nitrile and the process carried out with it. The catalyst is a nano-powder with nano-crystallite sizes of 5-30 nm or 10-40 nm (minimum and maximum Feret diameter), which consists of aggregates with a median of about 24 $\mu$m. For industrial use, however, it is necessary to recover the catalyst at the end of the reaction for recycling, e.g., by filtering. In principle, the cross-flow filtration described in patent application WO 2020/161067 A1 would be suitable for industrial application. However, this procedure requires high pumping capacities, which makes its industrial implementation disadvantageous.

A classic, comparatively inexpensive filtration technology, such as e.g., strip filters or filter cartridges, is not practicable contrary to the examples in EP 3199519 A1 and CN 112608261 A since in aqueous solution the aggregates (median 24 $\mu$m) break down over time to smaller agglomerates (median 0.4 $\mu$m) due to friction on pipe walls and mechanical energy input by the pumps used. The latter particle size range cannot be mapped by classical filtrations. One might expect that the extrusion of $CeO_2$ nano-powders might be a suitable way for preparing shaped catalysts. However, Example 3.3 below shows that extruded catalysts (e.g., those produced in Example 2.1 by extrusion of a $CeO_2$ nano-powder) give a low yield and a low selectivity for the formation of methionine by hydrolysis of methionine nitrile. Therefore, extruded catalysts are not suitable for use in the direct production of methionine by hydrolysis of methionine nitrile.

Accordingly, there was still a need for a catalyst for the direct production of amino acids, e.g., methionine, by hydrolysis of amino nitriles, e.g., methionine nitrile, and/or amino amides, e.g., methionine amide, on industrial scale.

It was found that this problem is solved in that granular $CeO_2$ comprising catalysts for the hydrolysis of amino nitriles and/or amino amides to amino acids are prepared by the steps of subjecting a $CeO_2$ comprising powder and at least one liquid binder to high shear wet granulation, followed by drying and/or calcinating of the thus obtained granulates, and sieving the dried and/or calcined granulates to a particle size from 100 to 5000 $\mu$m.

One object of the present invention is therefore a process for preparing a granular $CeO_2$ comprising catalyst for the hydrolysis of an amino nitrile and/or amino amide to an amino acid or derivative thereof, comprising the steps of

a) subjecting a $CeO_2$ comprising powder and a liquid binder to a high shear wet granulation to provide granulates,
b) drying and/or calcinating the granulates obtained from step a), and
c) sieving the dried and/or calcinated granulates obtained from step b) to a particle size from 100 to 5000 $\mu$m to give the granular $CeO_2$ comprising catalyst.

In principle, any $CeO_2$ comprising powder can be used in the preparation of the granular catalysts according to the present invention, provided that said $CeO_2$ comprising powder has a sufficient or the desired catalytic activity in the hydrolysis of an amino nitrile, e.g., methionine nitrile, and or amino amide, e.g., methionine amide, to the corresponding amino acid, e.g., methionine. For example, the $CeO_2$ comprising material disclosed in EP 3199519 A1, WO 2020/161067 A1, or WO 2020/249495 A1 can be used in the preparation of the granular catalyst according to the present invention.

In principle, the $CeO_2$ comprising powder is also not subject to any limitation regarding its size and shape, provided that size and shape of the $CeO_2$ comprising powder are suitable for high shear wet granulation. Preferably, the $CeO_2$ comprising powder is a nano-powder.

In one embodiment of the process according to the present invention the $CeO_2$ comprising powder comprises $CeO_2$ comprising particles with a BET surface area of from 35 +/- 10% to 300 +/- 10% $m^2/g$ measured according to DIN ISO 9277-5 (2003), a mean maximum Feret diameter $x_{Fmax, mean}$ of from 3 +/- 10% to 40 +/- 10% nm and mean minimum Feret diameter $x_{Fmin, mean}$ of from 2 +/- 10% to 30 +/- 10% nm, both measured according to DIN ISO 9276-6 (2012).

In a preferred embodiment of the process according to the present invention the $CeO_2$ comprising powder comprises $CeO_2$ comprising particles with a BET surface area of from 35 +/- 10% to 65 +/-10% $m^2/g$ measured according to DIN ISO 9277-5 (2003), a mean maximum Feret diameter $x_{Fmax, mean}$ of from 10 +/- 10% to 40 +/- 10% nm and mean minimum Feret diameter $x_{Fmin, mean}$ of from 5 +/-10% to 30 +/- 10% nm, both measured according to DIN ISO 9276-6 (2012).

In another preferred embodiment of the process according to the present invention the $CeO_2$ comprising powder comprises $CeO_2$ comprising particles with a BET surface area of from 175 +/-10% +/- 10% to 300 +/- 10% $m^2/g$ or from 225 +/- 10% to 265 +/- 10% measured according to DIN ISO 9277-5 (2003), a mean maximum Feret diameter $x_{Fmax, mean}$ of from 3 +/- 10% to 40 +/- 10% nm and mean minimum Feret diameter $x_{Fmin, mean}$ of from 2+/- 10% to 30 +/- 10% nm, both measured according to DIN ISO 9276-6 (2012).

Preferably, the $CeO_2$ comprising powder comprises from 50 to 100 wt.-% of $CeO_2$. For example, the $CeO_2$ comprising particles comprise 50 wt.-%, 70 wt.-%, 80 wt.-% or 100 wt.-% of $CeO_2$, and the remainder being one or more metals or metal oxides different from cerium or cerium oxides, such as $ZrO_2$. For example, the $CeO_2$ comprising particle can be a mixture of $CeO_2$ with $ZrO_2$ and/or $TiO_2$, such as $(CeO_2)_{0.5+x}(ZrO_2)_{0.5-x}$, e.g., $(CeO_2)_{0.5}$-$(ZrO_2)_{0.5}$, $(CeO_2)_{0.6}$-$(ZrO_2)_{0.4}$, $(CeO_2)_{0.7}$-$(ZrO_2)_{0.3}$, $(CeO_2)_{0.8}$-$(ZrO_2)_{0.2}$ or $(CeO_2)_{0.9}$-$(ZrO_2)_{0.1}$, or $(CeO_2)_{0.5+x}(TiO_2)_{0.5-x}$, e.g., $(CeO_2)_{0.5}$-$(TiO_2)_{0.5}$, $(CeO_2)_{0.6}$-$(TiO_2)_{0.4}$, $(CeO_2)_{0.7}$-$(TiO_2)_{0.3}$, $(CeO_2)_{0.8}$-$(TiO_2)_{0.2}$ or $(CeO_2)_{0.9}$-$(TiO_2)_{0.1}$, in each case with x ranging from 0 to 0.5, e.g., 0, 0.1, 0.2, 0.3, 0.4, or 0.5. Nevertheless, it is particularly preferred that the $CeO_2$ comprising particles comprise 100 wt.-% of $CeO_2$. In principle, it is also possible that a mixture of a $CeO_2$ comprising powder comprising 100 wt.-% of $CeO_2$ and a $CeO_2$ comprising powder comprising $CeO_2$, $ZrO_2$ and/or $TiO_2$, preferably comprising $CeO_2$ and $ZrO_2$, is used in the process according to the present invention.

Preferably, the mean minimum Feret diameter $x_{Fmin, mean}$ of the $CeO_2$ comprising particles is always smaller than the mean maximum Feret diameter $x_{Fmax, mean}$ of said particles.

Preferably, the $CeO_2$ comprising particles have a mean aspect ratio $x_{Fmin, mean}$ / $x_{Fmax, min}$ of from 0.55 to 0.85, measured according to DIN ISO 9276-6 (2012).

It is further preferred that the $CeO_2$ comprising particles comprises from 50 to 100 wt.-% of $CeO_2$, have a BET surface area of from 35 to 65 $m^2/g$ measured according to DIN ISO 9277-5 (2003), a mean maximum Feret diameter $x_{Fmax, mean}$ of from 10 to 40 nm and a mean minimum Feret diameter $x_{Fmin, mean}$ of from 5 to 30 nm, both measured according to DIN ISO 9276-6 (2012), and a mean aspect ratio $x_{Fmin, mean}$ / $x_{Fmax, min}$ of from 0.55 to 0.85, measured according to DIN ISO 9276-6 (2012).

Preferably, the $CeO_2$ comprising particles have a mean aspect ratio $x_{Fmin, mean}$ / $x_{Fmax, min}$ of from 0.55 +/- 10% to 0.80 +/- 10%, measured according to DIN ISO 9276-6 (2012).

It is further preferred that the $CeO_2$ comprising powder comprises $CeO_2$ comprising particles with a BET surface area of from 175 +/- 10% +/- 10% to 300 +/- 10% $m^2/g$ or from 225 +/- 10% to 265 +/-10% measured according to DIN ISO 9277-5 (2003), a mean maximum Feret diameter $x_{Fmax, mean}$ of from 3 +/- 10% to 40 +/- 10% nm and mean minimum Feret diameter $x_{Fmin, mean}$ of from 2+/- 10% to 30 +/- 10% nm, both measured according to DIN ISO 9276-6 (2012), and a mean aspect ratio $x_{Fmin, mean}$ / $x_{Fmax, min}$ of from 0.55 +/- 10% to 0.80 +/- 10%, measured according to DIN ISO 9276-6 (2012).

Preferably, the $CeO_2$ comprising particles have a lattice plane distance of from 0.24 +/- 10% to 0.32 +/- 10% nm.

Preferably, the $CeO_2$ comprising particles comprise octahedral particles.

Preferably, the $CeO_2$ comprising particles have a mean equivalent circular diameter $x_{A, mean}$ of from 5 to 45 nm, measured according to DIN ISO 9276-6 (2012).

It is further preferred that the $CeO_2$ comprising particles comprise from 50 to 100 wt.-% of $CeO_2$, have a BET surface area of from 35 to 65 $m^2/g$ measured according to DIN ISO 9277-5 (2003), a mean maximum Feret diameter $x_{Fmax, mean}$ of from 10 to 40 nm and a mean minimum Feret diameter $x_{Fmin, mean}$ of from 5 to 30 nm, both measured according to DIN ISO 9276-6 (2012), a mean aspect ratio $x_{Fmin, mean}$ / $x_{Fmax, min}$ of from 0.55 to 0.85, measured according to DIN ISO 9276-6 (2012), and a mean equivalent circular diameter $x_{A, mean}$ of from 5 to 45 nm, measured according to DIN ISO 9276-6 (2012).

Preferably, the $CeO_2$ comprising particles have a mean equivalent circular diameter $x_{A, mean}$ of from 3 +/- 10% to 30 +/- 10% nm, measured according to DIN ISO 9276-6 (2012).

It is further preferred that the $CeO_2$ comprising powder comprises $CeO_2$ comprising particles with a BET surface area of from 175 +/- 10% +/- 10% to 300 +/- 10% $m^2/g$ or from 225 +/- 10% to 265 +/-10%, measured according to DIN ISO 9277-5 (2003), a mean maximum Feret diameter $x_{Fmax, mean}$ of from 3 +/- 10% to 40 +/- 10% nm and mean minimum Feret diameter $x_{Fmin, mean}$ of from 2+/- 10% to 30 +/- 10% nm, both measured according to DIN ISO 9276-6 (2012), a mean aspect ratio $x_{Fmin, mean} / x_{Fmax, min}$ of from 0.55 +/- 10% to 0.80 +/- 10%, measured according to DIN ISO 9276-6 (2012), and a mean equivalent circular diameter $x_{A, mean}$ of from 3 +/- 10% to 30 +/- 10% nm, measured according to DIN ISO 9276-6 (2012).

Preferably, the $CeO_2$ comprising particles have a crystallinity of at least 50 %. It is further preferred that the $CeO_2$ comprising particles have a crystallinity of from 50 to 100%, of from 50 to 95%, or of from 55 to 90%.

In the context of the present invention the term high shear wet granulation is used as known to the person skilled in the art and denotes a granular product development technology, which typically involves the following steps: (a) Addition and dry mixing of the material powders, (b) addition of a liquid powder (either a binder solution or solvent) into the powder mixtures at a lower impeller and chopper speed, and (c) wet massing with both the impeller and chopper running at high speed (this can be varied depending on the product).

In principle, the high shear wet granulation in step a) of the process according to the present invention can be performed in any suitable device for granulating, provided it leads to the $CeO_2$ comprising catalysts according to the present invention. The equipment used for performing the high shear wet granulation in step a) is a typical high shear wet granulator or a mixer or granulator suitable for this purpose. In principle, the step a) of the process according to the present invention is not subject to any limitation regarding a specific design of the used high shear wet granulator, mixer, or granulator, provided, that it leads to the granular $CeO_2$ comprising catalysts according to the present invention. For example, some high shear wet granulators mainly consist of a mixing bowl, an impeller, and an auxiliary chopper. The function of the impeller is to mix the powder and to promote granular densification. The function of the chopper is to break apart the wet lumps or promote the growth of smaller particles. Other mixers or granulators suitable as high shear wet granulators comprise a mixing tank equipped with a rotating and inclined mixing pan, an eccentrically arranged mixing tool and a scraper fixed on the wall or bottom. The eccentrically arranged mixing tool can be a pin-type rotator, with several cylindrical or wedge shaped pins, extending along the rotational axis of the stirring tool. During stirring the pins describe circles, surrounding coaxially the rotational axis. Using such a stirring tool facilitates the maintenance of a homogeneous liquid phase inside the mixer. The powder is typically provided in the mixing tank or mixing bowl of the granulator or mixer. Then, the impeller or the mixing tank is rotated with a given speed, e.g., in the range of from 60 to 120 rounds per minute, depending on the individual design of the mixer or granulator, with the chopper or the mixing tool, preferably pin-type rotator, being rotated in the opposite direction to the impeller or the mixing tank, e.g., with a circulation speed of 2 to 10 m/s, for 60 to 120 seconds. The binder solution is added in portions, e.g., a first portion of 85% and a second portion of 15%, via one or more nozzles into the mixer or granulator over a total time of from 60 to 240 seconds, with the circulation speed of the chopper or mixing tool being increased to 5 to 20 m/s circulation speed. Then the tank or bowl wall is cleaned with a rubber wiper. Subsequently, the second portion of the binder solution is added over 30 to 120 seconds at a similar circulation of the chopper or mixing tool as the first portion of the binder solution. Then the tank or bowl wall is cleaned again with a rubber wiper. The thus obtained mass is granulated at a circulation of the chopper or mixing tool of from 20 to 60 m/s for 10 to 650 seconds. Next, the thus obtained wet granulate is subjected to drying and/or calcination in step b).

In principle, the drying in step b) of the process according to the present invention is not subject to any limitations regarding its conduct, in particular regarding the time period and the temperature at which it is performed, provided these conditions provide for a drying of the granulates obtained from step a) of the process. Nevertheless, it is preferred to perform the drying of the granulates obtained from step a) at temperatures of at least 100 °C, e.g., from 100 to 200 °C, for at least one hour, e.g., from 1 to 5 hours.

Also, the calcination in step b) of the process according to the present invention is in principle not subject to any limitations regarding its conduct, in particular regarding the time period and the temperature at which it is performed, provided these conditions provide for a calcination of the granulates obtained from step b) of the process. Preferably, the calcination in step b) is performed at a temperature in the range of from 400 to 900 °C, in particular in the range of from 400 to 600 °C, in a time range of from 1 to 10 hours, preferably in a time range of from 3 to 8 hours. For example, the calcination can be performed using a temperature program with a predetermined heating rate, e.g., of 5 °C per minute, until the final temperature, e.g., a temperature of 500 °C, is reached. The final temperature is maintained for a determined time range, e.g., 5 hours, followed by a cooling to room temperature within a determined time range, e.g., one hour.

The sieving in step c) is not subject to any limitations. Preferably, sieving of the granulates to a target particle size range in step c) is performed using an appropriate sieving machine. In detail, two sieve bottoms are used to get granulates in the target particle size range: a sieve bottom having a mesh size representing the upper limit and a sieve bottom having a mesh size representing the lower limit. First, the sieve bottom having a mesh size representing the upper limit, e.g., a mesh size of 1000 $\mu$m, is applied to the sieving machine and the portion is sift out, that has a larger particle size than desired, here

> 1000 $\mu$m. Next, the granulates that went through that sieve are subjected to a second sieving, now with the sieve bottom representing the lower limit, e.g., a mesh size of 500 $\mu$m. The sieving material retained in this sieve bottom represents the target sieving fraction, here a fraction with a particle size from 500 to 1000 $\mu$m.

In a further embodiment of the process according to the present invention the dried and/or calcinated granulates obtained from step b) are sieved in step c) to a particle size from 125 to 500 $\mu$m, from 500 to 1000 $\mu$m, from 1000 to 2500 $\mu$m and/or from 2500 to 5000 $\mu$m.

The fractions outside the target particle size range, e.g., < 500 $\mu$m and > 1000 $\mu$m, are sift out in step c). This, however, does not mean that those granulates are lost. Rather, it is preferred to subject them, at least the larger granulates, to a grinding step to obtain a $CeO_2$ comprising powder, and to subject this powder again to the process according to the present invention.

Preferably, the $CeO_2$ comprising powder has a density of from 0.05 +/- 10% to 1.2 +/- 10% g/mL. In case the $CeO_2$ comprising powder is a mixture of a $CeO_2$ comprising powder comprising 100 wt.-% of $CeO_2$ and a $CeO_2$ comprising powder comprising $CeO_2$, $ZrO_2$ and/or $TiO_2$, said density denotes the density of the mixture.

In the context of the present invention the term binder is used equivalent to the term binding agent and denotes a substance that holds other materials, here the $CeO_2$ comprising powder, together to form a cohesive whole mechanically, chemically, by adhesion or cohesion.

A liquid binder as used in the process according to the present invention comprises one or more binders and a granulating liquid. In principle, the at least one binder of the process according to the present invention is not subject to any limitation regarding its composition and the number of components, e.g., the specific binder and the granulating liquid.

Therefore, said liquid binder may comprise an inorganic binder and/or an organic binder. Good results were obtained when said liquid binder comprises an organic binder, e.g., one or more organic binders, specifically when the binder itself is an organic binder.

In one embodiment of the process according to the present invention the liquid binder comprises an organic binder.

Suitable organic binders comprise natural polymers, synthetic polymers, or sugars, which act as the glue connecting the powder together. Examples for natural polymers comprises sugars such as polysaccharide, commercially available as zusoplast PS1. Examples for synthetic polymers comprise polyvinyl alcohol, e.g., commercially available as mowiol 8-88, polyvinylpyrrolidone, commercially available as PVP K30, and cellulose ethers such as hydroxyethylmethylcellulose (HEMC), for examples various tyloses, e.g., commercially available as tylose MH 1000.

During the calcination the one or more organic binders is/are burnt without residue and form the desired pores and surfaces in the granular structure.

The granulating liquid can be water or an alcohol, which allow for a good solubility of all types of useful inorganic or organic binders. However, it is preferred that the granulating liquid is water, from the point of view of working safety and process simplicity.

In another embodiment of the process according to the present invention the liquid binder comprises water as granulating liquid.

The process according to the present invention opens the way to specific catalysts for the hydrolysis of an amino nitrile and/or amino amide to the corresponding amino acid or derivative thereof on industrial scale.

Another object of the present invention is therefore a granular $CeO_2$ comprising catalyst for the hydrolysis of an amino nitrile and/or amino amide to an amino acid or derivative thereof obtained by a process according to the present invention and/or having

- a BET surface area of from 70 +/- 10% to 130 +/- 10% $m^2$/g measured according to DIN ISO 9277-5 (2003), and
- a particle size from 100 to 5000 $\mu$m.

In the context of the present invention, diameters d larger than 4 nm, i.e., 4 nm < d < 400 $\mu$m, were chosen as representative for the pore volume of macro- and mesopores and the diameters smaller than 1 $\mu$m, i.e., 4 nm < d < 1 $\mu$m, were chosen as representative for the pore volume of smaller macropores as well as mesopores of the catalyst according to the present invention.

Preferably, the catalyst according to the present invention has a pore volume 4 nm < d < 400 $\mu$m of from 0.3 +/- 10% to 1.2 +/- 10% mL/g, and/or a pore volume 4 nm < d < 1 $\mu$m of from 0.1 +/- 10% to 0.7 +/- 10% mL/g

In an embodiment the catalyst according to the present invention has a particle size from 125 to 500 $\mu$m, from 500 to 1000 $\mu$m, from 1000 to 2500 $\mu$m and/or from 2500 to 5000 $\mu$m.

Preferably, the catalyst according to the present invention has a d50 value of from 400 +/- 10% to 2000 +/- 10% $\mu$m, measured according to ISO 13322-1:2004 and determined according to ISO 9276.

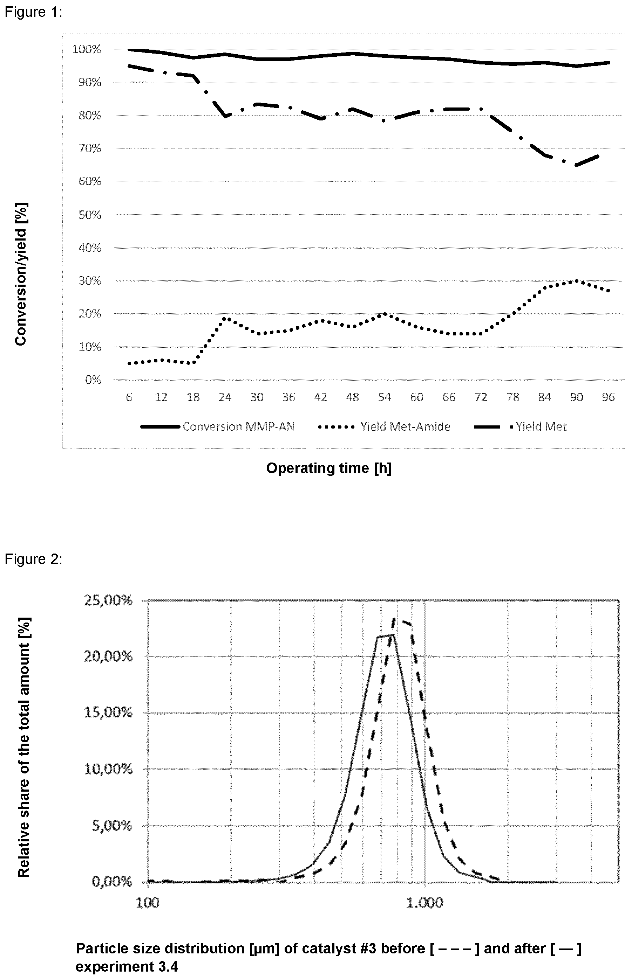

The catalysts according to the present invention and the catalysts obtained by the process according to the present invention are also characterized by an excellent mechanic stability. This is the result of an exemplary comparison of the particle size distribution of the catalyst #3 according to the present invention, determined prior and after the use of said catalyst in a fixed-bed reactor for reacting amino nitrile to a mixture comprising the corresponding amino amide and the corresponding amino acid, here reacting methionine nitrile to a mixture comprising methionine amide and methionine (see example 3.4), for a total time of 100 hours. Figure 2 shows the particle size distribution of catalyst #3 prior to its use and after its use in the preparation of methionine for a total time of 100 hours. Compared to its particle size distribution before the testing, there are no significant changes in the particle size distribution of catalyst #3 after its use. This shows that the catalyst particles are stable under the synthesis conditions applied in this testing. In detail, this means that the catalysts according to the present invention are also characterized by a low abrasion, preferably, an abrasion of from 5 +/- 0.5 to 12 +/- 1 %. Abrasion of the catalysts is determined using a sieving machine, for example AS200 (Retsch GmbH), with a sieve bottom, for example having a mesh size of 500 $\mu$m. A weighted quantity of the already pre-sieved granulates, for example 10 g, is placed on the sieve bottom and subjected to sieving in continuous operation at a set frequency for a given time, for example at frequency of 70 Hz for 6 minutes. After sieving has been completed, the sieve bottoms are removed from the sieving machine and the final weight is determined for the coarse material of the granulates, that was retained by the sieve bottom having the mentioned mesh size, here a mesh size of 500 $\mu$m. Abrasion on a percentual basis is calculated using the formula:

$$Abrasion[\%] = \frac{10.00\ g - m(final\ weight)[g]}{10.00\ g}$$

In one embodiment the catalyst according to the present invention has an abrasion of from 5 +/- 0.5 to 12 +/- 1 %.

In another embodiment the catalyst according to the present invention comprises from 50 to 100 wt.-% of $CeO_2$.

The bulk density of the catalysts according to the present invention varies depending on their specific composition. For example, catalysts comprising 100 wt.-% of $CeO_2$, may have a bulk density in the range of from 0.05 to 0.4 g/mL, preferably 0.1 to 0.2 g/mL. In case, where the catalysts comprise more than 50 wt.-% but less than 100 wt.-% of $CeO_2$, e.g., 30 wt.-% of $TiO_2$, 10 or 15 wt.-% of $ZrO_2$, the remainder being $CeO_2$, the bulk density may vary from 0.05 to 2.0 g/mL or 0.1 to 1.5 g/mL or 1.0 to 1.5 g/mL. In terms of the particles, a lower bulk density typically leads to larger surface areas of the particles, and a higher bulk density typically leads to larger mechanic stability of the particles. Both parameters typically influence each other in opposite directions. Therefore, bulk densities of from 0.05 to 2.0 g/mL or 0.1 to 1.5 g/mL or 1.0 to 1.5 g/mL for the catalysts according to the present invention are considered a good balance between a large surface area of the catalyst particles (typically achieved by a lower bulk density) and a high mechanic stability of the catalyst particles (typically achieved by a higher bulk density).

It is preferred that the catalyst according to the present invention have a bulk density in the range of from 0.05 to 2.0 mg/mL, preferably in the range of from 0.1 to 1.5 g/mL.

The bulk density of the granular catalysts is determined using a measuring cylinder with a volume of 50 mL. That cylinder is placed on an analytical balance, filled with the granulates obtained from sieving to target particle size range until a volume of 50 mL is reached and then the final weight is read from the analytical balance. The bulk density of the material in question is obtained by means of the formula:

$$bulk\ density \left[\frac{g}{mL}\right] = \frac{m(granulates)[g]}{50\ mL}$$

Both the catalysts obtained by the process according to the present invention and the catalysts according to the present invention can be used in the hydrolysis of an amino nitrile and/or an amino amide to an amino acid.

A further object of the present invention is therefore a method for the preparation of an amino acid or derivative thereof comprising the steps of contacting a solution or suspension comprising an amino nitrile and/or an amino amide with water in the presence of a catalyst obtained by the process according to the present invention and/or in the presence of a catalyst according to the present invention.

In the context of the present invention the term amino acid is used as known to the person skilled in the art and denotes an organic compound containing an amino group (-$NH_2$) and a carboxyl group (-COOH) along with a side chain specific to each amino acid, wherein the amino group may be also present in protonated form as -$NH_3^+$ and the carboxyl group may be also present in deprotonated form as carboxylate -$COO^-$. The term amino acid comprises the D-enantiomer and the L-enantiomer of the amino acid in question as well as the racemic mixture of both enantiomers, often also referred to as the corresponding D,L-amino acid.

In the context of the present invention the term amino acid derivative or the use of the term derivative thereof in context with an amino acid is used to denote any analogue compounds of the amino acid in question, where the amino acid is substituted with a functional group or moiety different from the amino group, or where a functional group in the side chain of the amino acid is replaced with a different functional group or moiety. For example, the amino group of methionine may be replaced with a hydroxy group in 2-hydroxy-4-(methylthio)butanoic acid, also known as the hydroxy analogue of methionine or methionine hydroxy analogue, abbreviated as MHA, or the hydroxy group of tyrosine may be replaced with the 4-hydroxy-3,5-diiodphenoxy moiety in thyroxin, 2-amino-3-[4-(4-hydroxy-3,5-diiodphenoxy)-3,5-diiodphenyl]propanic acid. The term amino acid derivative also comprises the D-enantiomer and the L-enantiomer of the amino acid derivative in question as well as the racemic mixture of both enantiomers, often also referred to as the corresponding D,L-amino acid derivative.

In the context of the present invention the term amino nitrile and amino amide are used to denote the corresponding starting compounds whose hydrolysis leads to the desired amino acid or derivative thereof. For example, when the amino acid to be prepared is methionine, the corresponding amino nitrile is methionine nitrile, and the corresponding amino amide is methionine amide. For example, when the amino acid derivative to be prepared is 2-hydroxy-4-(methylthio)butanoic acid, the corresponding amino nitrile is 2-hydroxy-4-(methylthio)butane nitrile, and the corresponding amino amide is 2-hydroxy-4-(methylthio)butane amide.

The hydrolysis of an amino nitrile to the corresponding amino acid or derivative thereof always proceeds via the intermediate stage of the corresponding amino amide. For example, if the hydrolysis reaction starts from methionine nitrile, it always proceeds via the intermediate stage of methionine amide, and finally gives the desired methionine. Hence, the method according to the present invention allows for the preparation of an amino acid or derivative thereof starting from the corresponding preceding amino nitrile, from the corresponding preceding amino amide, or from a mixture of the amino nitrile and the amino amide in any conceivable ratio to each other. It is clear without saying that when a specific enantiomer of an amino acid or derivative thereof is to be prepared by the method according to the present invention, the hydrolysis starts from the specific enantiomer of the corresponding amino nitrile and/or amino amide. For example, if L-methionine is to be prepared in the method according to the present invention, the hydrolysis starts from L-2-amino-4-(methylthio) butane nitrile and/or L-2-amino-4-(methylthio)butane amide.

In principle, the method according to the present invention is not subject to any limitations regarding the preparation of a specific amino acid or derivative thereof. Rather, any conceivable amino acid or derivative thereof can be prepared in the method according to the present invention, provided that there is access to or availability of the corresponding amino nitrile and/or amino amide. Nevertheless, it is preferred to prepare an alpha-amino acid or derivative thereof in the method according to the present invention.

In the context of the present invention the term alpha-amino acid is used as known to the person skilled in the art and denotes the amino acids containing an amino group bonded directly to the alpha-carbon, i.e., the carbon atom next to the carboxyl group. This term includes proline and hydroxyproline, which are secondary amines. Alpha-amino acids are therefore the most abundant amino acids in nature and most relevant amino acids in terms for nutrition and growth of humans and animals. Specifically, 22 of the alpha-amino acids are called biogenic or proteinogenic amino acids because they are the building blocks of proteins - sometimes they are therefore also called essential amino acids.

In the context of the present invention the term alpha-amino acid derivative or the use of the term derivative thereof in context with an alpha-amino acid is used to denote any analogue compounds of the alpha-amino acid in question, where the alpha-amino acid is substituted with a functional group or moiety different from the amino group, or where a functional group in the side chain of the alpha-amino acid is replaced with a different functional group or moiety. For example, the amino group of methionine may be replaced with a hydroxy group in 2-hydroxy-4-(methylthio)butanoic acid, also known as the hydroxy analogue of methionine or methionine hydroxy analogue, abbreviated as MHA, or the hydroxy group of tyrosine may be replaced with the 4-hydroxy-3,5-diiodphenoxy moiety in thyroxin, 2-amino-3-[4-(4-hydroxy-3,5-diiodphenoxy)-3,5-diiodphenyl]propanoic acid. The term amino acid derivative also comprises the D-enantiomer and the L-enantiomer of the amino acid derivative in question as well as the racemic mixture of both enantiomers, often also referred to as the corresponding D,L-amino acid derivative.

In an embodiment of the method according to the present invention the amino acid to be prepared is an alpha-amino acid, or a derivative thereof.

In the context of the present invention the term alpha-amino nitrile and alpha-amino amide are used to denote the corresponding starting compounds whose hydrolysis leads to the desired alpha-amino acid or derivative thereof. For example, when the alpha-amino acid to be prepared is methionine, the corresponding alpha-amino nitrile is 2-amino-4-(methylthio)butane nitrile, herein often called methionine nitrile or MMP-AN, and the corresponding alpha-amino amide is 2-amino-4-(methylthio)butane amide, herein often called Met-amide.

In principle, any conceivable alpha-amino acid or derivative thereof can be prepared from the corresponding alpha-amino nitrile and/or alpha-amino amide in the method according to the present invention. Nevertheless, it is preferred that the alpha-amino acid to be prepared is methionine, lysine, threonine, tryptophan, valine, or a derivative of any of these because these are the commercially most relevant alpha-amino acids, at least from a nutritional point of view.

In one embodiment of the method according to the present invention the alpha-amino acid to be prepared is methionine, lysine, threonine, tryptophan, valine, or a derivative of any of these.

Since the catalysts according to the present invention and the catalysts obtained by the process according to the present invention are characterized by an excellent mechanic stability, it is conceivable to use them in any type of reactor suitable for carrying out a heterogeneously catalyzed reaction, for example in a reactor with a stationary filling of the catalyst according to the present invention, such as a fixed-bed reactor, in a reactor with a moving bed of said catalyst, i.e., a moving bed reactor, or in a reactor with fluidized bed of said catalyst, i.e. a fluidized-bed reactor. Nevertheless, it is preferred that the method according to the present invention is performed in a fixed-bed reactor because this reactor design allows to keep the mechanical stress on the granular catalyst as low as possible.

Preferably, the method according to the present invention is performed in a fixed-bed reactor.

In principle, the method according to the present invention is not subject to any limitation regarding its design. Depending on the yields and the selectivity for the formation of the desired amino acids or derivative thereof, the method according to the present invention may be performed in a one-time run, a two-time run or even a multiple-time run at the same or different reaction conditions in each run, wherein in the case of a two-time or multiple-time run the reaction output of one stage, e.g., the first stage reaction output, is the reaction input for the next stage, e.g., the reaction input for the second stage.

For example, it is beneficial to perform the method according to the present invention in a two-time run: In the first stage an aqueous solution of the amino nitrile, e.g., methionine nitrile, is reacted in a fixed-bed reactor filled with the catalyst according to the present invention and/or the catalyst obtained by the process according to the present invention to a mixture of the amino amide, e.g., methionine amide, and the amino acid or derivative thereof, e.g., methionine, followed in the second or further stage by reacting the thus obtained mixture in a another fixed-bed reactor filled also with the catalyst according to the present invention and/or the catalyst obtained by the process according to the present invention to the desired amino acid or derivative thereof. The first and second or further reactors may be filled with the same catalyst, i.e., a catalyst according to the present invention having the same composition and/or the same characteristics. Preferably, the second or further stage is carried out at a higher temperature than the first stage in order to reach full conversion of the starting material. Preferably, the first stage is carried out at a temperature of up to 90 °C, e.g., from 50 to 90 °C, and the second or further stage is carried out at a temperature of up to 150 °C, e.g., from 90 to 150 °C. This allows to obtain the desired amino acid or derivative thereof quite efficiently.

It is preferred that the method according to the present invention is carried out at temperature in the range of from 50 to 150 °C, preferably from 50 to 90 °C or from 90 to 150 °C.

It is also preferred that the method according to the present invention is carried out in two or more stages, wherein the reaction output of one stage, e.g., the first stage reaction output, is the reaction input for the next stage, e.g., the reaction input for the second stage.

It is further preferred that the method according to the present invention is carried out in two or more stages, wherein the reaction output of one stage, e.g., the first stage reaction output, is the reaction input for the next stage, e.g., the reaction input for the second stage, with the next stage being carried out at a higher temperature, e.g., 90 to 150 °C, than the previous stage, e.g., 50 to 90 °C.

The space-time-yield and the amount of the catalyst used are important factors for the industrial feasibility: The use of the granular catalysts according to the present invention and of the granular catalysts obtained by the process according to the present invention allows to obtain rates of the weight hourly space velocity (WHSV, i.e., the mass of the amino nitrile used per hour and per mass of granular $CeO_2$ comprising catalyst) of from 0.05 to 10 $h^{-1}$, preferably 0.2 to 6 $h^{-1}$, with high conversion and high selectivity at the same time. The latter directly describes the activity of the granular catalyst because insufficient/reduced activity of the catalyst may result in an increased residence time of the starting compound, here for example the amino nitrile. This however would favor the thermal decomposition of the starting material and lead to side-reactions, such as the formation of dinitriles and the formation of secondary products, which would in the worst case decrease yield and selectivity for the desired main acids or derivative significantly.

It is therefore preferred that the method according to the present invention is carried out at a weight hourly space velocity of from 0.05 to 10 $h^{-1}$.

Preferably, the concentration of the amino nitrile in the method according to the present invention ranges from 5 to 20 wt.-%.

Preferably, the pressure in the method according to the present invention ranges from 1 to 10 bar.

The present invention is further illustrated by the following examples and figures.

**Description of the Figures:**

**Figure 1** shows the progress of the conversion and the yields in the example 3.4 being continuously operated at T = 80 °C and WHSH = 0.6 $h^{-1}$ for 96 h. The solid black line (-) at the top represents the conversion of 2-amino-4-(methylthio)butane nitrile (methionine nitrile, MMP-AN), the broken black line (- • -) in the middle represents the yield for 2-amino-4-(methylthio)butane amide (Met-amide), and the dotted line (• • •) at the bottom represents the yield for methionine (Met).

**Figure 2** shows the particle size distribution of the catalyst #3 before and after its use in example 3.4 at T = 80 °C, WHSH = 0.6 h$^{-1}$ for 100 h. The broken black line (- -) represents the particle size distribution of catalyst #3 prior to its use in the testing and the solid black line (-) represents the particle size distribution of catalyst #3 after its use in the testing for a total time of 100 hours.

**Examples:**

**1. Analytical methods**

**1.1 HPLC-chromatography:**

Chromatographic analyses of 2-hydroxy-4-(methylthio)butane nitrile (MMP-CN), 2-amino-4-(methylthio)butane nitrile (methionine nitrile, MMP-AN), 2-amino-4-(methylthio)butane amide (methionine amide, Met-amide), 3-(methylthio) propionaldehyde (MMP), and methionine (Met) were performed using HPLC systems from JASCO or Agilent with an RP-18 column (250 x 4,6 mm; 5 $\mu$m) and a subsequent UV detection at 210 nm. A mixture consisting of 3.3 g $H_3PO_4$, 6.8 g $CH_3CN$, and 89.9 g $H_2O$ was used as eluent with a flow of 1 mL/min. 10 $\mu$L of the respective sample solution (50 mg sample in 25 mL $H_2O$) were injected into the eluent for analysis. Calibration was done in advance by injection of suitable standard stock solutions of the analyst and a subsequent comparison of peak areas with external standards as commonly done in organic chemical syntheses.

**1.2 BET surface area:**

The BET surface areas $A_{BET}$ were determined by physical adsorption of nitrogen on the surface of the solid and by calculating the amount of adsorbate gas corresponding to a monomolecular layer on the surface according to the Brunauer, Emmett, and Teller (BET) method. The samples used (0.2 - 0.9 g) were degassed at 150 °C for 20 min under vacuum prior to the measurement. The determination was then carried out at the temperature of liquid nitrogen (77 K). The amount of gas adsorbed was measured by a static-volumetric, 3-point measurement using a TriStar 3000 Micrometrics instrument. The method is described in general in DIN ISO 9277-5 (2003) and was applied accordingly.

**1.3 Determination of pore volume and mean pore diameter**

The pore volume and the mean pore diameter were determined according to DIN 66134 ($N_2$ sorption according to Barret, Joyner, Halenda) with respect to pore sizes between either 4 nm < d < 400 $\mu$m or between 4 nm < d < 1 $\mu$m.

**1.4 Thermogravimetric analyses (TGA)**

Thermogravimetric analyses (TGA) were performed under air at a heating rate of 5 K/min in the range from 25 to 500 °C.

**1.5 Particle size of granular catalysts**

The particle size of the granular catalysts was determined by means of optical analysis of 200 mL of each granular catalyst using a CCD camera in a Camsizer® (Retsch Technology GmbH) according to ISO 13322-1:2014 and the median d50 was determined according to ISO 9276.

**1.6 Particle size distribution of powders**

The particle size distribution of powders was determined by means of laser diffraction according to ISO 13320:2009 and the median d50 was determined according to ISO 9276. For this purpose, a spate point of the material was mixed with 10 mL water and 0.5 g/L tetrasodium pyrophosphate and the thus obtained mixture was subjected to laser diffraction using an LS 13320 laser diffraction spectrometer (Beckmann-Coulter) with Universal Liquid Module (ULM).

**1.7 Sieving of the granulates to target particle size range**

Sieving of the granulates to a target particle size range was performed using a sieving machine AS200DIGIT (Retsch GmbH). Two sieve bottoms were used to get granulates in the target particle size range: a sieve bottom having a mesh size representing the upper limit and a sieve bottom having a mesh size representing the lower limit. First, the sieve bottom having a mesh size representing the upper limit, e.g., a mesh size of 1000 $\mu$m, was applied to the machine and the portion was sieved, that had a larger particle size than desired, here > 1000 $\mu$m. Next, the granulates that went through that sieve was subjected to a second sieving, now with the sieve bottom representing the lower limit, e.g., a mesh size of 500 $\mu$m. The sieving material retained in this sieve bottom represents the target sieving fraction, here a fraction with a particle size from 500 to 1000 $\mu$m. The fractions outside the target particle size range, i.e., < 500 $\mu$m and > 1000 $\mu$m, were ground once more and then used for further granulation.

### 1.8 Determination of abrasion and bulk density of the granular catalysts

The abrasion of the granular catalysts was determined using a sieving machine AS200 (Retsch GmbH) with a sieve bottom having a mesh size of 500 $\mu$m. 10.00 g of the already pre-sieved granulates was placed on the sieve bottom and subjected to sieving in continuous operation at a frequency of 70 Hz for 6 minutes. After sieving has been completed, the sieve bottoms were removed from the sieving machine and the final weight was determined for the coarse material of the granulates, that was retained by the sieve bottom having a mesh size of 500 $\mu$m. Abrasion on a percentual basis was calculated using the formula:

$$Abrasion[\%] = \frac{10.00\ g - m(final\ weight)[g]}{10.00\ g}$$

The bulk density of the granular catalysts was determined using a measuring cylinder with a volume of 50 mL. That cylinder was placed on an analytical balance, filled with the granulates obtained from sieving to target particle size range until a volume of 50 mL was reached and then the final weight was read from the analytical balance. The bulk density of the material in question was obtained by means of the formula:

$$bulk\ density\ \left[\frac{g}{mL}\right] = \frac{m(granulates)[g]}{50\ mL}$$

### 2. Preparation of the catalytically active material

The $CeO_2$ powders used for the preparation of the granular catalysts according to the present invention have the characteristics disclosed in EP 3199519 A1, WO 2020/161067 A1 or WO 2020/249495 A1 regarding their composition, degree of crystallinity, lattice plane distance, BET surface area $A_{BET}$, mean maximum Feret diameter $x_{Fmax,mean}$, minimum Feret diameter $x_{Fmin,\ mean}$, mean aspect ratio $x_{Fmin,\ mean}$ / $x_{Fmax,\ mean}$, and mean equivalent circular diameter $X_{A,\ mean}$.

The other chemicals used in the catalyst preparation such as tylose MH 1000 (methylhydroxy ethylcellulose), zusoplast PS1 (polysaccharide), mowiol 8-88 (polyvinyl alcohol) or PVP K30 (polyvinyl pyrrolidone) were purchased from various distributors such as Sigma Aldrich and were used without additional pre-treatment.

### 2.1 Extrusion of a $CeO_2$ nano-powder (not according to the invention)

416.1 g $CeO_2$ nano-powder, 5 g zusoplast PS1 (Zschimmer & Schwarz) and 15 g tylose H 30000 P2 (Shin Etsu) were provided in a universal kneader with a trough volume of 2.5 L (LUK 2.5) and premixed with a kneader rotation speed of 40 rounds per minute and a reverse operating discharge screw conveyor having a rotation speed of 11 rounds per minute for 10 minutes. The trough was cooled over a cryostat to a temperature of 10 °C. Subsequently, desalinated water was added in portions of each 50 g directly after the pre-mixing, after a further minute, and after a further minute kneading time and the rotation speed of the reverse operating discharge screw conveyor was increased to 25 rounds per minute. After a further two minutes 8.8 g desalinated water was added and after further three minutes once more a further 6.7 g desalinated water was added to adjust the plasticity of the kneading mass. The kneading mass showed a good plastic deformability. Finally, 5 g zusoplast WE 8 (Zschimmer & Schwarz) were added after eight minutes to adjust the wall slippage of the mass.

Extrusion was started after 37 minutes of kneading time at the conditions described above. A nozzle with a 4 x 2.7 mm drillings was used for this. The kneader rotation speed was set to 40 rounds per minute and the mass was pressed through the nozzle by means of the now forward running screw conveyor at a rotation speed of 68 rounds per minute. Here, a pressure of 6.8 bar set in at the screw outlet. The extrudates coming out of the nizzle were cut with a wire granulator to wire lengths of 4 mm +/- 0.5 mm (rotation speed 400-600 rounds per minute, 2 wires).

The wet wires thus obtained were then pre-dried on a conveyor belt at 60 °C for 30 minutes and subsequently in air at room temperature overnight. The thus pre-dried extrudates were calcinated in a muffle furnace (Nabertherm LT15/11/P320). The following temperature program was used: heat-up rate 5 °C/min, final temperature 500 °C was maintained for 5 hours, and subsequently cooling to 25 °C within 1 hour.

The obtained extrudates had a length of 4 mm and a diameter of 2.7 mm (catalyst #1). They were analyzed as described in chapter 1, which gave the following results: $A_{BET} = 27 \ m^2/g$; pore volume 4 nm < d < 400 $\mu$m [mL/g] = 2.26; pore volume 4 nm < d < 1 $\mu$m = 0.21; abrasion = 7.23%; bulk density = 1.5 g/mL.

## 2.2 Granulation of a CeO$_2$ nano-powder according to the invention

100 g CeO$_2$ nano-powder according to table 1 was provided in an Eirich laboratory mixer EL 1 (Eirich Mischbehälter 1L). The laboratory mixer was equipped with a pin-type rotator. The mixing tank ran with on step 1 (85 rounds per minute) opposite to the rotation direction of the pin-type rotator. Prior to the addition of the binder solution, a CeO$_2$ nano-powder was mixed at 5 m/s circulation speed of the pin-type rotator for 80 seconds. Then 85% of the amount of the aqueous binder solution mentioned in table 1 were added at 10 m/s circulation speed of the pin-type rotator within 140 seconds. The tank wall was cleaned with a rubber wiper. Subsequently, the remaining 15 % of the amount of aqueous binder solution mentioned in table 1 were added at 10 m/s circulation speed of the pin-type rotator within 50 seconds. Then the tank wall was cleaned again with a rubber wiper. The thus obtained mass was granulated at 30 m/s circulation speed of the pin-type rotator for the time mentioned in table 1.

The obtained wet granulate was calcinated in muffle furnace (Nabertherm LT15/11/P320). The following temperature program was used: heat-up rate 5 °C/min, final temperature 500 °C was maintained for 5 hours, and subsequently cooling to 25 °C within 1 hour.

The final wight was 96.1 g and thus 96.1 % the theory. The obtained granulate was separated to the following sieving fractions by means of analysis sieve (Retsch) on a sieving machine AS 200 digit (Retsch):

| Fraction 1: | > 2500 $\mu$m | = 12.14 g = 12.63% |
| Fraction 2: | 1000-2500 $\mu$m | = 43.08 g = 43% |
| Fraction 3: | 500-1000 $\mu$m | = 24.25 g = 25.29% |
| Fraction 4: | 250-500 $\mu$m | = 11.50 g = 11.93% |
| Fraction 5: | 125-250 $\mu$m | = 4.52 g = 4.70% |
| Fraction 6: | < 125 $\mu$m | = 3.34 g = 2.41% |

| Catalyst # | Composition of the used CeO$_2$ nano-powder | Density of the used CeO$_2$ nano-powder | Used aqueous binder solution | Amount of binder solution [g] | Additional additive | Granulation time [s] | d50 prior to sieving [μm] |
|---|---|---|---|---|---|---|---|
| #2 | CeO$_2$ | 0.07 | Tylose MH 1000 (1.5 wt.-%) | 100 | - | 120 | 1594 |
| #3 | 70% CeO$_2$ 30% CeO$_2$-ZrO$_2$ | 1.06 0.32 | Tylose MH 1000 (1.5 wt.-%) | 36 | - | 30 | 7669 |
| #4 | 80% CeO$_2$ 20% CeO$_2$-ZrO$_2$ | 0.07 0.32 | Tylose MH 1000 (1.5 wt.-%) | 110 | - | 90 | 1295 |
| #5 | CeO$_2$ | 0.07 | Tylose MH 1000 (1.5 wt.-%) | 76 | - | 640 | - |
| #6 | CeO$_2$ | 0.07 | Tylose MH 1000 (1.5 wt.-%) | 190 | TiO$_2$ (Hombikat, d = 3.9 g/mL) | 10 | - |
| #7 | CeO$_2$ | 0.07 | Mowiol 8-88 1.0 wt.-% | 130 | - | 20 | 1671 |
| #7 | CeO$_2$ | 0.07 | PVP K30 3.0 wt.-% | 130 | - | 40 | 1380 |

Table 1: Preparation details of the used catalyst.

The target sieving fractions, 500-1000 μm and 1000-2500 μm, were analyzed for their physical characteristics as described in chapter 1, which gave the results summarized in table 2.

| Catalyst # | Composition of catalyst granulates | Sieving fraction [μm] | Abrasion [%] | Bulk density [g/mL] | Pore volume 4 nm < d < 400 μm [mL/g] | Pore volume 4 nm < d < 1 μm [mL/g] | $A_{BET}$ [m²/g] | d50 [μm] | Ignition loss [%] |
|---|---|---|---|---|---|---|---|---|---|
| #2 | $CeO_2$ | 500-1000 | 11.0 | 0.14 | 1.18 | 0.65 | 71 | 756 | 3.1 |
| #2 | $CeO_2$ | 1000-2500 | 7.8 | 0.19 | 0.92 | 0.68 | 73 | 1970 | 2.0 |
| #3 | $(CeO_2)_{0.85}(ZrO_2)_{0.15}$ | 500-1000 | 9.0 | 1.37 | 0.34 | 0.19 | 126 | 456 | 3.8 |
| #4 | $(CeO_2)_{0.9}(ZrO_2)_{0.1}$ | 500-1000 | 10.1 | 0.49 | - | - | - | - | - |
| #5 | $(CeO_2)_{0.7}(TiO_2)_{0.3}$ | 500-1000 | 6.1 | 0.69 | - | - | - | - | - |
| #6 | $CeO_2$ | 500-1000 | 7.4 | 0.34 | - | - | - | - | - |
| #7 | $CeO_2$ | 500-1000 | 8.5 | 0.31 | - | - | - | - | - |
| #8 | $CeO_2$ | 500-1000 | 11.6 | 0.38 | - | - | - | - | - |

Table 2: Physical characteristics of the prepared catalysts

## 3. Synthesis Examples

### 3.1 Synthesis of 2-amino-4-(methylthio)butane nitrile starting from 2-hydroxy-4-(methyl-thio)butane nitrile

10.1 g 2-hydroxy-4-(methylthio)butane nitrile (MMP-CN; 90 wt.-% in water, 69.3 mmol, 1 mol. eq.) were mixed with 26.0 g $NH_3$ (32 wt.-% in water, 7 mol. eq., 48.8 mmol) in a glass reactor and sealed subsequently. The slightly beige colored and turbid emulsion containing 25 wt.-% MMP-CN was stirred and heated to 50 °C for 30 minutes by means of a pre-heated water bath. The obtained light yellow solution was analyzed by HPLC chromatography confirming a 100% conversion of MMP-CN with a selectivity of 98.8% towards 2-amino-4-(methylthio)butane nitrile (MMP-AN; 67.2 mmol) and 2-amino-4-(methylthio)butane amide (Met-amide; 1.2 mmol). The MMP-AN solution thus obtained consisted of 8.75 g MMP-AN (67.2 mmol), 0.18 g Met-amide (1.2 mmol), 7.14 $NH_3$ (419 mmol, 6mol.eq.) and 19.9 g water.

### 3.2 Direct conversion of the obtained 2-amino-4-(methylthio)butane nitrile towards a mixture comprising 2-amino-4-(methylthio)butane amide and methionine

The MMP-AN solution obtained in example 3.1 consisted of 8.75 g MMP-AN (67.2 mmol), 0.18 g Met-amide (1.2 mmol), 7.14 $NH_3$ (419 mmol, 6mol. eq.) and 19.9 g water. A further 36.2 g water was added to this solution to set an MMP-AN concentration of 12 wt.-%, that was used in this example.

The conversion was performed in a vertically installed fixed bed reactor containing 3.0 g of the various catalyst granulates of the sieving fraction 500-1000 μm obtained according to example 2.2 and identified in table 3 below as used catalysts #2 to #8. For this purpose, the reaction solution obtained according to example 3.1 and having an MMP-AN concentration of 12 wt.-% was pumped with a customary double piston lifting pump, as typically used for HPLC devices, at a flow rate of 1.0 mL from above or below into said fixed bed reactor, which corresponds to a weight hourly space velocity of 2.4 $h^{-1}$. The fixed bed reactor was either heated with an electric jacket heating or a double wall oil heating using a thermostat. The internal temperature was monitored with a PT100 thermocouple and the heating was set up in such a way that a temperature of 70 °C was kept constant over the whole reaction. The reactor was operated at ambient pressure. The inner diameter was 10 mm. The reactor was started up with pure water and brought to the operating temperature before the feed was switched to the solution comprising 12 wt.-% of MMP-AN. After 2 h operating time the reaction solution was collected for analytics using HPLC-chromatography. The results are summarized in table 3 below.

| Used catalyst | Conversion of MMP-AN [%] | Yield Met + Met-amide [%] | Yield Met [%] | Ratio of yields Met : Met-amide | Selectivity Met +Met-amide [%] | Selectivity Met [%] |
|---|---|---|---|---|---|---|
| #2 | 94.3 | 92.7 | 48.1 | 1.1 | 98.4 | 50.9 |
| #3 | 86.9 | 84.2 | 49.2 | 1.4 | 96.9 | 56.2 |
| #4 | 94.1 | 94.1 | 56.3 | 1.4 | 100.0 | 59.7 |
| #5 | 85.3 | 81.1 | 35.0 | 0.8 | 96.3 | 40.5 |
| #6 | 85.3 | 81.7 | 36.2 | 0.8 | 95.8 | 42.0 |
| #7 | 97.4 | 97.4 | 58.8 | 1.5 | 100.0 | 60.3 |
| #8 | 96.5 | 95.8 | 55.3 | 1.4 | 99.3 | 57.2 |

Table 3: Results of the experiments 3.2 at a WHSV of 2.4 h$^{-1}$ and 70 °C.

**3.3 Direct conversion of the 2-amino-4-(methylthio)butane nitrile towards a mixture comprising 2-amino-4-(methylthio)butane amide and methionine at various particle sizes of the used granulates and at different weight hourly space velocities**

The granulated catalyst #2 was sieved to various particle size fractions as described in chapter 2.2 and used as mentioned in table 4 below. The extruded catalyst #1 prepared according to chapter 2.1 was used as comparison catalyst, see table 4 below.

As described in example 3.2 the catalysts were used in a fixed bed reactor and the reaction solution obtained from example 3.1 was set to the desired MMP-AN concentration as indicated in table 4 below by addition of the appropriate amount of water. The fixed bed reactor filled with the catalysts of different sieving fractions or even extruded catalysts was operated at a pressure of 10 bar that was set with a pressure retention valve. The, weight hourly space velocities and the internal temperature were adjusted as mentioned in table 4 below.

| Used catalyst | Concentration MMP-AN [wt.-%] | Sieving fraction [µm] | WHSV [h$^{-1}$] | Internal temperature [°C] | Conversion MMP-AN [%] | Yield Met + Met-amide [%] | Yield Met [%] | Ratio of yields Met : Met-amide | Selectivity Met + Met-amide [%] | Selectivity Met [%] |
|---|---|---|---|---|---|---|---|---|---|---|
| #1* | 5.9 | extrudate | 0.22 | 70 | 87.0 | 72.8 | 20.5 | 0.4 | 83.7 | 23.5 |
| #1* | 5.2 | extrudate | 1.0 | 70 | 30.9 | 28.1 | 16.4 | 1.1 | 90.8 | 44.6 |
| #2 | 5.8 | 500-1000 | 0.25 | 60 | 100 | 97.8 | 96.4 | 26.7 | 97.8 | 96.4 |
| #2 | 5.8 | 500-1000 | 0.9 | 60 | 100 | 93.0 | 81.6 | 4.4 | 93.0 | 81.6 |
| #2 | 5.8 | 500-1000 | 5.2 | 60 | 94.7 | 89.5 | 50.4 | 1.1 | 94.5 | 53.1 |
| #2 | 5.5 | 125-500 | 4.9 | 60 | 93.8 | 93.8 | 52.9 | 1.3 | 100 | 56.3 |
| #2 | 12.0 | 2500-5000 | 3.6 | 70 | 98.0 | 94.9 | 55.0 | 1.3 | 97.3 | 56.1 |
| #2 | 12.0 | 1000-2500 | 3.6 | 70 | 98.8 | 98.8 | 57.4 | 1.4 | 100 | 58.0 |
| #2 | 18.0 | 1000-2500 | 2.7 | 70 | 90.8 | 86.9 | 32.8 | 0.6 | 95.7 | 36.0 |
| #2 | 16.0 | 1000-2500 | 1.2 | 85 | 100 | 100 | 88.3 | 7.5 | 100 | 88.3 |

Table 4: results of the example 3.3 with different concentrations of 2-amino-4-(methylthio)butane nitrile (MMP-AN), catalysts of different sieving fractions or even extruded catalysts subjected to different weight hourly space velocities and different temperatures (* identifies comparison examples).

### 3.4 Direct conversion of the 2-amino-4-(methylthio)butane nitrile towards a mixture comprising 2-amino-4-(methylthio)butane amide and methionine

These experiments were performed in a fixed bed reactor as described in example 3.2 with a solution comprising 12 wt.-% of MMP-AN, with the exception that the experiments were performed at an internal temperature of 80 °C and a WHSV of 0.6 h$^{-1}$ for 96 hours in total. The catalyst #3 with a sieving fraction of 500 to 1000 µm according to table 1 was used as the $CeO_2$ catalyst granulate catalyst. The fixed bed reactor was operated at a pressure of 10 bar that was set with a pressure retention valve. After the experiment durations mentioned in table 5 below the product solution was collected separately and its composition, and resulting from this, the conversion of MMP-AN, the yield for Met-amide, the yield for Met and the selectivity for Met + Met-Amide were determined, which are summarized in table 5 below. The progress of the conversion and the yields are shown in Figure 1.

| Experiment duration [h] | Conversion MMP-AN [%] | Yield Met-amide [%] | Yield Met [%] | Selectivity Met + Met-amide [%] |
|---|---|---|---|---|
| 6 | 100 | 5 | 95 | 100 |
| 12 | 99 | 6 | 93 | 100 |
| 18 | 98 | 5 | 92 | 99 |
| 24 | 99 | 19 | 80 | 100 |
| 30 | 97 | 14 | 83 | 100 |
| 36 | 97 | 15 | 82 | 100 |
| 42 | 98 | 18 | 79 | 99 |
| 48 | 99 | 16 | 82 | 99 |
| 54 | 98 | 20 | 78 | 100 |
| 60 | 98 | 16 | 81 | 99 |
| 66 | 97 | 14 | 82 | 99 |
| 72 | 96 | 14 | 82 | 100 |
| 78 | 96 | 20 | 75 | 99 |
| 84 | 96 | 28 | 68 | 100 |
| 90 | 95 | 30 | 65 | 100 |
| 96 | 96 | 27 | 69 | 100 |

Table 5: Results of the continuously performed experiment (96 h, 80 °C, WHSV = 0.6 h$^{-1}$).

After 100 hours testing the catalyst was analyzed for any potential changes in the particle size distribution of the catalyst #3. The Figure 2 shows the particle size distribution of catalyst #3 prior to its use in the testing and after its use in the testing for a total time of 100 hours. Compared to its particle size distribution before the testing, there are no significant changes in the particle size distribution of catalyst #3 after its use in the testing for a total time of 100 hours. This shows that the catalyst particles are stable under the synthesis condition applied in this testing.

### 3.5 Reacting the obtained solution comprising 2-amino-4-(methylthio)butane nitrile and 2-amino-4-(methylthio)butane amide

This experiment used the reaction solution obtained with catalyst #2 as described in example 3.2 consisting of 0.7 wt.-% 2-amino-4-(methylthio)butane nitrile (MMP-AN), 6.8 wt.-% 2-amino-4-(methylthio)butane amide (Met-amide), 6.9 wt.-% methionine (Met), 10 wt.-% NH$_3$, and 76.3 wt.-% water.

This solution was subjected according to example 3.2 in a fixed bed reactor equipped with the catalyst #3 (see table 1), with the exception that an internal temperature of 100 °C and a WHSV of 0.25 h$^{-1}$ was set for a testing time of 78 hours in total (see table 6 below). The reactor operated at a pressure of 10 bar that was set with a pressure retention valve. After the experiment durations mentioned in table 6, the product solution was collected separately and its composition, and resulting from this, the conversion of MMP-AN, the yield for Met-amide, the yield for Met and the selectivity for Met + Met-Amide were determined, which are summarized in table 6 below.

| Duration of experiment [h] | Conversion MMP-AN [%] | Yield Met-amide [%] | Yield Met [%] | Selectivity Met + Met-Amide [%] |
|---|---|---|---|---|
| 2 | 100 | 10 | 90 | 100 |
| 4 | 100 | 10 | 90 | 100 |
| 24 | 100 | 12 | 88 | 100 |
| 33 | 100 | 10 | 90 | 100 |
| 50 | 100 | 12 | 88 | 100 |
| 78 | 100 | 10 | 90 | 100 |

Table 6: Results of the continuously performed experiment (78 h, 100 °C, WHSV = 0.25 h$^{-1}$).

**Claims**

1. A process for preparing a granular $CeO_2$ comprising catalyst for the hydrolysis of an amino nitrile and/or amino amide to an amino acid or derivative thereof, comprising the steps of

    a) subjecting a $CeO_2$ comprising powder and a liquid binder to a high shear wet granulation to provide granulates,
    b) drying and/or calcinating the granulates obtained from step a), and
    c) sieving the dried and/or calcinated granulates obtained from step b) to a particle size from 100 to 5000 $\mu$m to give the granular $CeO_2$ comprising catalyst.

2. The process according to claim 1, wherein the $CeO_2$ comprising powder comprises from 50 to 100 wt.-% of $CeO_2$, has a BET surface area of from 35 +/- 10% to 300 +/- 10% m$^2$/g measured according to DIN ISO 9277-5 (2003), a mean maximum Feret diameter $x_{Fmax, mean}$ of from 3 +/- 10% to 40 +/- 10% nm and mean minimum Feret diameter $x_{Fmin, mean}$ of from 2 +/- 10% to 30 +/- 10% nm, both measured according to DIN ISO 9276-6 (2012).

3. The process according to claim 2, wherein the $CeO_2$ comprising powder has a BET surface area of from 35 +/- 10% to 65 +/- 10% m$^2$/g measured according to DIN ISO 9277-5 (2003), a mean maximum Feret diameter $x_{Fmax, mean}$ of from 10 +/- 10% to 40 +/- 10% nm and mean minimum Feret diameter $x_{Fmin, mean}$ of from 5 +/- 10% to 30 +/- 10% nm, both measured according to DIN ISO 9276-6 (2012).

4. The process according to claim 2, wherein the $CeO_2$ comprising material has a BET surface area of from 175 +/- 10% +/- 10% to 300 +/- 10% m$^2$/g or from 225 +/- 10% to 265 +/- 10% measured according to DIN ISO 9277-5 (2003), a mean maximum Feret diameter $x_{Fmax, mean}$ of from 3 +/- 10% to 40 +/- 10% nm and mean minimum Feret diameter $x_{Fmin, mean}$ of from 2 +/- 10% to 30 +/- 10% nm, both measured according to DIN ISO 9276-6 (2012).

5. The process according to any of claims 1 to 4, wherein the $CeO_2$ comprising catalyst comprises from 50 to 100 wt.-% of $CeO_2$.

6. The process according to any of claims 1 to 5, wherein in step c) the dried and/or calcinated granulates obtained from step a) are sieved to a particle size from 125 to 500 $\mu$m, from 500 to 1000 $\mu$m, from 1000 to 2500 $\mu$m and/or from 2500 to 5000 $\mu$m.

7. The process according to any of claims 1 to 6, wherein the liquid binder comprises an organic binder.

8. The process according to any of claims 1 to 7, wherein the liquid binder comprises water as granulating liquid.

9. A granular $CeO_2$ comprising catalyst for the hydrolysis of an amino nitrile and/or amino amide to an amino acid or derivative thereof,
   obtained by a process according to any of claims 1 to 8.

10. The catalyst according to claim 9, wherein the catalyst has a particle size from 125 to 500 $\mu$m, from 500 to 1000 $\mu$m, from 1000 to 2500 $\mu$m and/or from 2500 to 5000 $\mu$m.

11. The catalyst according to claim 9 or 10, wherein the catalyst has a d50 value of from 400 +/-10% to 2000 +/- 10% $\mu$m, measured according to ISO 13322-1:2004 and determined according to ISO 9276.

12. The catalyst according to any of claims 9 to 11, wherein the catalyst has a pore volume 4 nm < d < 400 $\mu$m of from 0.3 +/- 10% to 1.2 +/- 10% mL/g and/or a pore volume 4 nm < d < 1 $\mu$m of from 0.1 +/- 10% to 0.7 +/- 10% mL/g

13. The catalyst according to any of claims 9 to 12, wherein the catalyst comprises from 50 to 100 wt.-% of $CeO_2$.

14. A method for the preparation of an amino acid or a derivative thereof comprising the step of contacting a solution or suspension comprising an amino nitrile and/or an amino amide with water in the presence of a catalyst obtained by the process according to any of claims 1 to 8 and/or in the presence of a catalyst according to any of claims 9 to 13.

15. The method according to claim 14, wherein the amino acid to be prepared is an alpha-amino acid, or a derivative thereof.


**Patentansprüche**

1. Verfahren zur Herstellung eines körnigen $CeO_2$ enthaltenden Katalysators für die Hydrolyse eines Aminonitrils und/oder Aminoamids zu einer Aminosäure oder einem Derivat davon, umfassend die Schritte

   a) Durchführen einer Nassgranulation mit hoher Scherung an einem $CeO_2$ enthaltenden Pulver und einem flüssigen Bindemittel unter Bereitstellung von Granulat,
   b) Trocknen und/oder Calcinieren des in Schritt a) erhaltenen Granulats und
   c) Sieben des aus Schritt b) erhaltenen getrockneten und/oder calcinierten Granulats auf eine Teilchengröße von 100 bis 5000 $\mu$m unter Erhalt des körnigen $CeO_2$ enthaltenden Katalysators.

2. Verfahren nach Anspruch 1, wobei das $CeO_2$ enthaltende Pulver 50 bis 100 Gew.-% $CeO_2$ umfasst und eine gemäß DIN ISO 9277-5 (2003) gemessene BET-Oberfläche von 35 +/- 10 % bis 300 +/- 10 % m$^2$/g, einen mittleren maximalen Feret-Durchmesser $X_{Fmax,mittel}$ von 3 +/- 10 % bis 40 +/- 10 % nm und einen mittleren minimalen Feret-Durchmesser $X_{Fmin,mittel}$ von 2 +/- 10 % bis 30 +/- 10 % nm, beide gemäß DIN ISO 9276-6 (2012) gemessen, aufweist.

3. Verfahren nach Anspruch 2, wobei das $CeO_2$ enthaltende Pulver eine gemäß DIN ISO 9277-5 (2003) gemessene BET-Oberfläche von 35 +/- 10 % bis 65 +/- 10 % m$^2$/g, einen mittleren maximalen Feret-Durchmesser $X_{Fmax,mittel}$ von 10 +/- 10 % bis 40 +/- 10 % nm und einen mittleren minimalen Feret-Durchmesser $X_{Fmin,mittel}$ von 5 +/-10 % bis 30 +/- 10 % nm, beide gemäß DIN ISO 9276-6 (2012) gemessen, aufweist.

4. Verfahren nach Anspruch 2, wobei das $CeO_2$ enthaltende Material eine gemäß DIN ISO 9277-5 (2003) gemessene BET-Oberfläche von 175 +/- 10 % +/- 10 % bis 300 +/- 10 % m$^2$/g oder von 225 +/- 10 % bis 265 +/- 10 %, einen mittleren maximalen Feret-Durchmesser $X_{Fmax,mittel}$ von 3 +/- 10 % bis 40 +/- 10 % nm und einen mittleren minimalen Feret-Durchmesser $X_{Fmin,mittel}$ von 2 +/- 10 % bis 30 +/- 10 % nm, beide gemäß DIN ISO 9276-6 (2012) gemessen, aufweist.

5. Verfahren nach einem der Ansprüche 1 bis 4, wobei der $CeO_2$ enthaltende Katalysator 50 bis 100 Gew.-% $CeO_2$ umfasst.

6. Verfahren nach einem der Ansprüche 1 bis 5, wobei in Schritt c) das aus Schritt a) erhaltene getrocknete und/oder calcinierte Granulat auf eine Teilchengröße von 125 bis 500 $\mu$m, von 500 bis 1000 $\mu$m, von 1000 bis 2500 $\mu$m und/oder von 2500 bis 5000 $\mu$m gesiebt wird.

7. Verfahren nach einem der Ansprüche 1 bis 6, wobei das flüssige Bindemittel ein organisches Bindemittel umfasst.

8. Verfahren nach einem der Ansprüche 1 bis 7, wobei das flüssige Bindemittel Wasser als Granulierflüssigkeit umfasst.

9. Körniger $CeO_2$ enthaltender Katalysator für die Hydrolyse eines Aminonitrils und/oder Aminoamids zu einer Aminosäure oder einem Derivat davon,

erhalten durch ein Verfahren nach einem der Ansprüche 1 bis 8.

10. Katalysator nach Anspruch 9, wobei der Katalysator eine Teilchengröße von 125 bis 500 $\mu$m, von 500 bis 1000 $\mu$m, von 1000 bis 2500 $\mu$m und/oder von 2500 bis 5000 $\mu$m aufweist.

11. Katalysator nach Anspruch 9 oder 10, wobei der Katalysator einen gemäß ISO 13322-1:2004 gemessenen und gemäß ISO 9276 bestimmten d50-Wert von 400 +/- 10 % bis 2000 +/- 10 % $\mu$m aufweist.

12. Katalysator nach einem der Ansprüche 9 bis 11, wobei der Katalysator ein Porenvolumen 4 nm < d < 400 $\mu$m von 0,3 +/- 10 % bis 1,2 +/- 10% ml/g und/oder ein Porenvolumen 4 nm < d < 1 $\mu$m von 0,1 +/-10 % bis 0,7 +/-10 % ml/g aufweist.

13. Katalysator nach einem der Ansprüche 9 bis 12, wobei der Katalysator 50 bis 100 Gew.-% $CeO_2$ umfasst.

14. Verfahren zur Herstellung einer Aminosäure oder eines Derivats davon, umfassend den Schritt des Kontaktierens einer Lösung oder Suspension, die ein Aminonitril und/oder ein Aminoamid umfasst, mit Wasser in Gegenwart eines durch das Verfahren nach einem der Ansprüche 1 bis 8 erhaltenen Katalysators und/oder in Gegenwart eines Katalysators nach einem der Ansprüche 9 bis 13.

15. Verfahren nach Anspruch 14, wobei es sich bei der herzustellenden Aminosäure um eine alpha-Aminosäure oder ein Derivat davon handelt.

## Revendications

1. Procédé de préparation d'un catalyseur comprenant du $CeO_2$ granulaire pour l'hydrolyse d'un amino nitrile et/ou d'un amino amide en un aminoacide ou un dérivé correspondant, comprenant les étapes de

   a) soumission d'une poudre comprenant du $CeO_2$ et d'un liant liquide à une granulation humide à cisaillement élevé pour fournir des granulés,
   b) séchage et/ou calcination des granulés obtenus à l'étape a), et
   c) tamisage des granulés séchés et/ou calcinés obtenus à l'étape b) jusqu'à une taille de particule de 100 à 5 000 $\mu$m pour donner le catalyseur comprenant du $CeO_2$ granulaire.

2. Procédé selon la revendication 1, dans lequel la poudre comprenant du $CeO_2$ comprend de 50 à 100 % en poids de $CeO_2$, a une surface spécifique BET de 35 $\pm$ 10 % à 300 $\pm$ 10 % $m^2$/g mesurée selon la norme DIN ISO 9277-5 (2003), un diamètre de Feret maximum moyen $x_{Fmax, moyen}$ de 3 $\pm$ 10 % à 40 $\pm$ 10 % nm et un diamètre de Feret minimum moyen $x_{Fmin, moyen}$ de 2 $\pm$ 10 % à 30 $\pm$ 10 % nm, les deux mesurés selon la norme DIN ISO 9276-6 (2012).

3. Procédé selon la revendication 2, dans lequel la poudre comprenant du $CeO_2$ a une surface spécifique BET de 35 $\pm$ 10 % à 65 $\pm$ 10 % $m^2$/g mesurée selon la norme DIN ISO 9277-5 (2003), un diamètre de Feret maximum moyen $X_{Fmax, moyen}$ de 10 $\pm$ 10 % à 40 $\pm$ 10 % nm et un diamètre de Feret minimum moyen $X_{Fmin, moyen}$ de 5 $\pm$ 10 % à 30 $\pm$ 10 % nm, les deux mesurés selon la norme DIN ISO 9276-6 (2012).

4. Procédé selon la revendication 2, dans lequel le matériau comprenant du $CeO_2$ a une surface spécifique BET de 175 $\pm$ 10 % à 300 $\pm$ 10 % $m^2$/g ou de 225 $\pm$ 10 % à 265 $\pm$ 10 % mesurée selon la norme DIN ISO 9277-5 (2003), un diamètre de Feret maximum moyen $X_{Fmax, moyen}$ de 3 $\pm$ 10 % à 40 $\pm$ 10 % nm et un diamètre de Feret minimum moyen $X_{Fmin, moyen}$ de 2 $\pm$ 10 % à 30 $\pm$ 10 % nm, les deux mesurés selon la norme DIN ISO 9276-6 (2012).

5. Procédé selon l'une quelconque des revendications 1 à 4, dans lequel le catalyseur comprenant du $CeO_2$ comprend de 50 à 100 % en poids de $CeO_2$.

6. Procédé selon l'une quelconque des revendications 1 à 5, dans lequel dans l'étape c), les granulés séchés et/ou calcinés obtenus dans l'étape a) sont tamisés jusqu'à une taille de particule de 125 à 500 $\mu$m, de 500 à 1 000 $\mu$m, de 1 000 à 2 500 $\mu$m et/ou de 2 500 à 5 000 $\mu$m.

7. Procédé selon l'une quelconque des revendications 1 à 6, dans lequel le liant liquide comprend un liant organique.

**8.** Procédé selon l'une quelconque des revendications 1 à 7, dans lequel le liant liquide comprend de l'eau comme liquide de granulation.

**9.** Catalyseur comprenant du $CeO_2$ granulaire pour l'hydrolyse d'un amino nitrile et/ou d'un amino amide en un aminoacide ou un dérivé correspondant, obtenu par un procédé selon l'une quelconque des revendications 1 à 8.

**10.** Catalyseur selon la revendication 9, dans lequel le catalyseur a une taille de particule de 125 à 500 $\mu$m, de 500 à 1 000 $\mu$m, de 1 000 à 2 500 $\mu$m et/ou de 2 500 à 5 000 $\mu$m.

**11.** Catalyseur selon la revendication 9 ou 10, dans lequel le catalyseur a une valeur de d50 de 400 $\pm$10 % à 2 000 $\pm$10 % $\mu$m, mesurée selon la norme ISO 13322-1:2004 et déterminée selon la norme ISO 9276.

**12.** Catalyseur selon l'une quelconque des revendications 9 à 11, dans lequel le catalyseur a un volume de pores de 4 nm < d < 400 $\mu$m allant de 0,3 $\pm$10 % à 1,2 $\pm$10 % mL/g et/ou un volume de pores de 4 nm < d < 1 $\mu$m allant de 0,1 $\pm$10 % à 0,7 $\pm$10 % mL/g

**13.** Catalyseur selon l'une quelconque des revendications 9 à 12, dans lequel le catalyseur comprend 50 à 100 % en poids de $CeO_2$.

**14.** Procédé pour la préparation d'un aminoacide ou d'un dérivé correspondant comprenant l'étape de mise en contact d'une solution ou suspension comprenant un amino nitrile et/ou un amino amide avec de l'eau en présence d'un catalyseur obtenu par le procédé selon l'une quelconque des revendications 1 à 8 et/ou en présence d'un catalyseur selon l'une quelconque des revendications 9 à 13.

**15.** Procédé selon la revendication 14, dans lequel l'aminoacide à préparer est un alpha-aminoacide, ou un dérivé correspondant.

Figure 1:

Operating time [h]

Figure 2:

Particle size distribution [μm] of catalyst #3 before [ – – – ] and after [ — ] experiment 3.4

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- US 5770769 A
- WO 2001060788 A1
- JP 3093753 A
- JP 3093754 A
- EP 3199519 A1
- WO 2018021338 A
- WO 2020161067 A1
- WO 2020161074 A1
- CN 112608261 A
- WO 2020249495 A1